# EUROPEAN PATENT SPECIFICATION

(11) **EP 0 345 457 B1**
(45) Date of publication and mention of the grant of the patent: **04.08.1993**
(21) Application number: 89107897.4
(22) Date of filing: 02.05.1989
(51) Int. Cl.: C07C 69/612, C07C 67/14, A61K 31/215

(54) **Antinflammatory agent for the therapy of prostatic hypertrophy, a process for the preparation thereof and pharmaceutical compositions containing it**
Entzündungshemmendes Mittel zur Behandlung der krankhaften Prostataerweiterung, ein Verfahren für seine Herstellung und dieses enthaltende pharmazeutische Zusammensetzungen
Agent antiinflammatoire pour le traitement de l'hypertrophie prostatique, un procédé pour sa préparation et compositions pharmaceutiques le contenant

(30) Priority: 13.05.1988 IT 2057188
(43) Date of publication of application: 13.12.1989
(73) Proprietor: PULITZER ITALIANA S.r.l., 00156 Roma (IT)
(72) Inventor: Rainoldi, Angelo, I-00156 Rome (IT)
(74) Representative: Minoja, Fabrizio

(56) References cited:
- FR-A- 2 336 126

## Description

The present invention relates to docosanyl 2-(p-isobutyl-phenyl)-propionate of formula I:

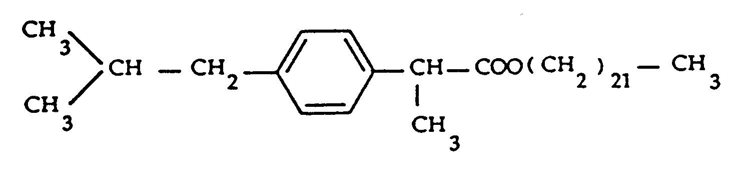

The present invention also relates to a process for the preparation of compound I and to pharmaceutical compositions containing it as the active ingredient.

Compound I is the ester of 2-(p-isobutyl-phenyl)-propionic acid, or Ibuprofen, which is well known and widely used in therapy due to the antiinflammatory and antiphlogistic activities thereof, with docosanol, a long chain alcohol which is present in vegetal extracts used for the treatment of prostatic adenoma.

GB. patent 1.459.233 discloses pharmaceutical compositions consisting of higher alkanols for the treatment of prostatic diseases and generally claims also the esters of said alkanols, but no examples of said esters are reported therein.

Now it has been found that compound I, which hereinafter will be defined also PU 3097, allows to obtain particularly favourable therapeutic results, which cannot directly be assigned to the properties of the two constituents of the molecule according to the invention.

Therefore, a main object of the invention is provided by pharmaceutical compositions for the treatment of disorders related to prostatic hypertrophy conditions.

The compositions of the invention, which can be prepared by means of conventional excipients and methods, can be administered orally, rectally or parenterally, in form of capsules, tablets, syrups, granulates, suspensions, suppositories and the like, containing 100 to 1500 mg of PU 3097.

A further object of the present invention concerns the use of docosanyl 2-(p-isobutyl-phenyl)-propionate for the preparation of a medicament having autiphlogistic and antiinflammatory activity

The daily dosage will obviously depend on many factors, but generally it will range from 500 to 5000 mg/day, optionally divided in more administrations.

PU 3097 is prepared by means of well known esterification techniques. For example, a reactive derivative of Ibuprofen, such as the acid chloride, is reacted with docosanol in inert solvents, in the presence of acid binding agents.

The following example further illustrates the invention without limiting it.

### EXAMPLE

4.4 g (0.02 mole) of 2-(p-isobutyl-phenyl)propionic acid chloride dissolved in 60 ml of dichloromethane were added with 2.4 ml (0.03 mole) of pyridine. 6.6 g (0.02 mole) of docosanol dissolved in 70 ml of dichloromethane were added to the reaction mixture, under stirring.

The reaction was slightly exothermic. The mixture was left to react for 12 hours at room temperature, under stirring.

The reaction was checked by means of thin layer chromatography (TLC), using 1:1 hexane-dichloromethane as the eluent mixture. chromatography (TLC), using 1:1 hexane-dichloromethane as the eluent mixture.

At the end of the reaction the mixture was washed with water, the layers were separated and the organic layer was treated with 20 ml of diluted HCI; the layers were separated again and the organic one was washed with water, then the solvent was distilled off under vacuum and the residue was purified on silica gel, using 4:1 hexane-dichloromethane as the eluent.
9 g of a solid compound were obtained, melting at 45 _{°} -46 _{°} C.
IR (NUJOL^{R}, cm-¹): 1740,850
NMR (CDCIa, TMS,6): 0.7-1.7 (50, m); 1.5 (3, d); 2.45 (2, d);3,65 (1, q);4.0 (2, t); 6.9-7.2 (4, m).

## Claims (Claims for the following Contracting State(s) : BE, CH, DE, FR, GB, IT, LI, LU, NL, SE, AT)

1. Docosanyl 2-(p-isobutyl-phenyl)-propionate of formula I

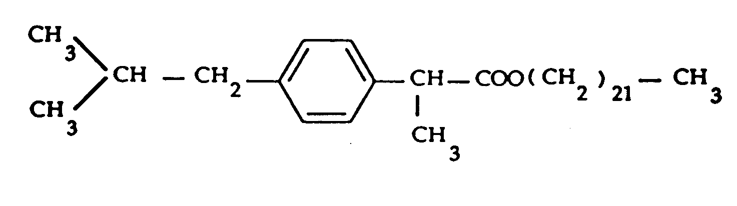

2. A process for the preparation of the compound of claim 1, which consists in reacting docosanol with a 2-(p-isobutyl-phenyl)-propionic acid reactive derivative.

3. A process as claimed in claim 2, in which the reactive derivative is the acid chloride.

4. Pharmaceutical compositions containing as the active ingredient the compound of claim 1.

5. The use of docosanyl 2-(p-isobutyl-phenyl)-propionate for the preparation of an antiinflammatory and antiphlogistic medicament. Claims for the following Contracting States: ES, GR

1. A process for the preparation of docosanyl 2-(p-isobutyl-phenyl)-propionate of formula I 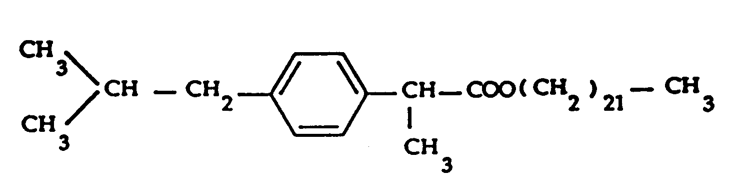 which consists in reacting docosanol with a 2-(p-isobutyl-phenyl)-propionic acid reactive derivative.

2. A process as claimed in claim 1, in which the reactive derivative is the acid chloride.

## Patentansprüche (Patentansprüche für folgende(n) Vertragsstaat(en) : BE, CH, DE, FR, GB, IT, LI, LU, NL, SE, AT)

1. 2-(p-lsobutyl-phenyl)-propionsäuredocosanylester der Formel I

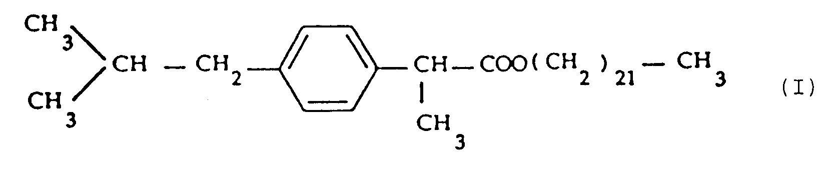

2. Verfahren zur Herstellung der Verbindung nach Anspruch 1, dadurch gekennzeichnet, daß man Docosanol mit einem reaktiven Derivat der 2-(p-lsobutylphenyl)-propionsäure umsetzt.

3. Verfahren nach Anspruch 2, dadurch gekennzeichnet, daß das reaktive Derivat das Säurechlorid ist.

4. Pharmazeutische Zusammensetzungen, dadurch gekennzeichnet, daß diese als wirksamen Bestandteil die Verbindung nach Anspruch 1 enthalten.

5. Verwendung von 2-(p-lsobutyl-phenyl)-propionsäuredocosanylester zur Herstellung eines antiinflammatorischen und antiphlogistischen Arzneimittels.

## Patentansprüche (Patentansprüche für folgende(n) Vertragsstaat(en) : ES, GR)

1. Verfahren zur Herstellung von 2-(p-lsobutyl-phenyl)-propionsäuredocosanylester der Formel I 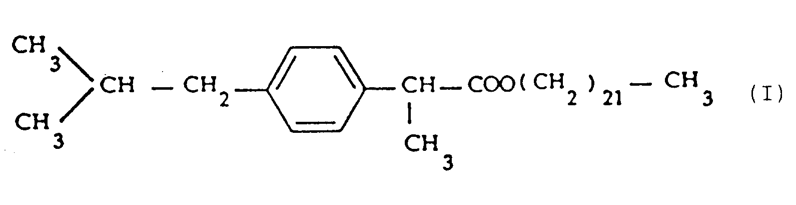 dadurch gekennzeichnet, daß man Docosanol mit einem reaktiven Derivat der 2-(p-lsobutyl-phenyl)-propionsäure umsetzt.

2. Verfahren nach Anspruch 1, dadurch gekennzeichnet, daß das reaktive Derivat das Säurechlorid ist.

## Revendications (Revendications pour l'(les) Etat(s) contractant(s) suivant(s) : BE, CH, DE, FR, GB, IT, LI, LU, NL, SE, AT)

1. (p-lsobutyl-phényl)-2 propionate de docosanyle de formule (I) :

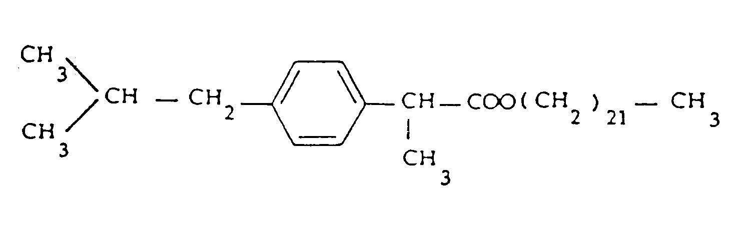

2. Procédé pour la préparation du composé de la revendication 1, qui consiste à faire réagir le docosanol avec un dérivé réactif de l'acide (p-isobutyl-phényl)-2 propionique.

3. Procédé selon la revendication 2, dans lequel le dérivé réactif est le chlorure d'acide.

4. Compositions pharmaceutiques contenant en tant qu'ingrédient actif le composé de la revendication 1.

5. Utilisation du (p-isobutyl-phényl)-2 propionate de docosanyle pour la préparation d'un médicament antiinflammatoire et antiphlogistique.

## Revendications (Revendications pour l'(les) Etat(s) contractant(s) suivant(s) : ES, GR)

1. Procédé de préparation du (p-isobutylphényl)-2 propionate de docosanyle de formule (I) : 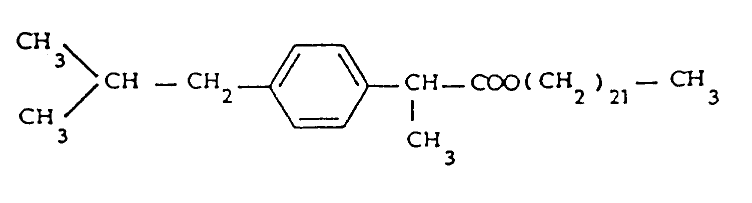 qui consiste à faire réagir le docosanol avec un dérivé réactif de l'acide (p-isobutyl-phényl)-2 propionique.

2. Procédé selon la revendication 1, dans lequel le dérivé réactif est le chlorure d'acide.
